(19) **Europäisches Patentamt / European Patent Office / Office européen des brevets**

(11) **EP 4 737 573 A1**

(12) **EUROPEAN PATENT APPLICATION**
published in accordance with Art. 153(4) EPC

(43) Date of publication:
06.05.2026 Bulletin 2026/19

(21) Application number: 24832133.3

(22) Date of filing: 28.06.2024

(51) International Patent Classification (IPC):
*C12N 15/62* (2006.01)   *A01N 63/50* (2020.01)
*A01P 3/00* (2006.01)   *A61K 38/47* (2006.01)
*A61K 47/18* (2017.01)   *A61P 11/00* (2006.01)
*A61P 27/02* (2006.01)   *A61P 31/04* (2006.01)
*C07K 14/005* (2006.01)   *C07K 19/00* (2006.01)
*C12N 15/12* (2006.01)   *C12N 15/33* (2006.01)

(52) Cooperative Patent Classification (CPC):
A01N 63/50; A01P 3/00; A61K 38/47; A61K 47/18;
A61P 11/00; A61P 27/02; A61P 31/04;
C07K 14/005; C07K 14/435; C07K 19/00;
C12N 15/62

(86) International application number:
PCT/JP2024/023622

(87) International publication number:
WO 2025/005276 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.06.2023 JP 2023108915

(71) Applicants:
• National University Corporation Okayama
University
Kita-ku,
Okayama-shi,
Okayama 700-8530 (JP)
• National University Corporation
Kochi University
Kochi-shi, Kochi 780-8520 (JP)

(72) Inventors:
• UCHIYAMA, Jumpei
Okayama-shi, Okayama 700-8530 (JP)
• UCHIYAMA, Iyo
Okayama-shi, Okayama 700-8530 (JP)
• FUKUDA, Ken
Kochi-shi, Kochi 780-8520 (JP)
• YAMASHIRO, Kenji
Kochi-shi, Kochi 780-8520 (JP)

(74) Representative: Müller-Boré & Partner
Patentanwälte PartG mbB
Friedenheimer Brücke 21
80639 München (DE)

Remarks:
The complete document including Reference Table(s) and the Sequence Listing(s) can be downloaded from the EPO website

(54) **POLYPEPTIDE PERMEABLE THROUGH OUTER MEMBRANE OF GRAM-NEGATIVE BACTERIUM, ANTIBACTERIAL PROTEIN COMPRISING SAID POLYPEPTIDE, ANTIBACTERIAL AGENT OR DISINFECTANT, PHARMACEUTICAL COMPOSITION, AND ANTIBACTERIAL AGENT COMPOSITION OR DISINFECTANT COMPOSITION**

(57) The present invention provides a polypeptide as defined in one of (i) to (iii) below, as well as an antibacterial protein, an antibacterial agent, a disinfectant, a pharmaceutical composition, an antibacterial agent composition, and a disinfectant composition, each comprising the following polypeptide:
(i) a polypeptide comprising the amino acid sequence represented by SEQ ID NO: 1;

(ii) a polypeptide comprising an amino acid sequence in which one or more amino acid residues are deleted, substituted, inserted, and/or added in the amino acid sequence represented by SEQ ID NO: 1; or
(iii) a polypeptide comprising an amino acid sequence having sequence identity of 80% or higher with the amino acid sequence represented by SEQ ID NO: 1.

EP 4 737 573 A1

## Description

Technical Field

The present invention relates to a polypeptide capable of permeating an outer membrane of Gram-negative bacteria, as well as an antibacterial protein, an antibacterial agent or disinfectant, a pharmaceutical composition, and an antibacterial composition or disinfectant composition, each containing the polypeptide.
In the present specification, the "polypeptide capable of permeating a lipopolysaccharide-containing outer membrane of Gram-negative bacteria" is sometimes abbreviated as "outer membrane-permeable polypeptide."

Background Art

*Pseudomonas aeruginosa* is a pathogenic bacterium that is inherently drug-resistant and that acquires drug resistance, causing intractable infections in humans and animals (such as cattle and dogs). The bacterium is also included in WHO's list of drug-resistant bacteria of high emergency importance. Today, there is a need to develop a novel antibacterial agent against *Pseudomonas aeruginosa.*
Investigation on conventional antibiotics, which are low-molecular-weight compounds, is continuing due to the presence of established development frameworks.
However, the library of low-molecular-weight compounds is limited, making it very difficult to discover anti-infective drugs with novel mechanisms of action. Given this situation, antimicrobial peptides, phages, and endolysins are currently being investigated as entirely different and novel modalities for anti-infective drugs.
Endolysins have been investigated, with the focus on natural-phage endolysins.
PTL 1 discloses an endolysin protein targeting staphylococci.
NPL 1 to NPL 3 each disclose an endolysin protein against Gram-negative bacteria.
Basically, the cell wall of Gram-negative bacteria includes an outer membrane, and a natural endolysin alone cannot kill the bacteria. Meanwhile, a natural endolysin capable of killing such Gram-negative bacteria does not have the bactericidal effect necessary and sufficient for drugs to treat infectious diseases.

Citation List

Patent Literature

PTL 1: JP2021-112152A

Non-patent Literature

NPL 1: Lai WCB, Chen X, Ho MKY, Xia J, Leung SSY. Bacteriophage-derived endolysins to target Gram-negative bacteria. Int J Pharm. 589, 119833, 2020.
NPL 2: De Maesschalck V, Gutierrez D, Paeshuyse J, Lavigne R, Briers Y. Advanced engineering of third-generation lysins and formulation strategies for clinical applications. Crit Rev Microbiol. 46(5), 548-564, 2020.
NPL 3: Gontijo MTP, Jorge GP, Brocchi M. Current Status of Endolysin-Based Treatments against Gram-negative Bacteria. Antibiotics (Basel). 10(10), 1143, 2021.

Summary of Invention

Technical Problem

One aim of the present invention is to provide an antibacterial agent or disinfectant having efficacy against Gram-negative bacteria, including multidrug-resistant bacteria, particularly *Pseudomonas aeruginosa.*
Another aim of the present invention is to provide a polypeptide capable of increasing the outer membrane permeability of Gram-negative bacteria to endolysin, and a polynucleotide encoding the polypeptide.

Solution to Problem

The present invention provides an outer membrane-permeable polypeptide, an antibacterial protein, a polynucleotide encoding the polypeptide and the protein, an antibacterial agent or disinfectant, a pharmaceutical composition, and an antibacterial composition or disinfectant composition.

(1) A polypeptide capable of permeating a lipopolysaccharide-containing outer membrane of Gram-negative bacteria, as defined in one of (i) to (iii) below:

(i) a polypeptide comprising the amino acid sequence represented by SEQ ID NO: 1;

```
SEQ ID NO: 1:

NSGTPKNVSRGTSSTKTTPKYKVKNGDNLTKIAKKHNTTVATLLKLNPGIKDPNMIRVGQTLNVT

GSGGKTHKVKSGDTLSKIAVDNKTTVSKLMNLNPEITNPNHIKVGQTIRLSRKLRRLKRKIAHKV

KKY;
```

(ii) a polypeptide comprising an amino acid sequence in which one or more amino acid residues are deleted, substituted, inserted, and/or added in the amino acid sequence represented by SEQ ID NO: 1, and capable of permeating a lipopolysaccharide-containing outer membrane of Gram-negative bacteria; or
(iii) a polypeptide comprising an amino acid sequence having sequence identity of 80% or higher with the amino acid sequence represented by SEQ ID NO: 1, and capable of permeating a lipopolysaccharide-containing outer membrane of Gram-negative bacteria.

(2) A polynucleotide as defined in one of (iv) to (vi) below:

(iv) a polynucleotide comprising a DNA sequence encoding the polypeptide according to (1);
(v) a polynucleotide comprising the DNA sequence represented by SEQ ID NO: 2:

```
SEQ ID NO: 2:

AATTCTGGCACGCCTAAGAACGTATCGCGCGGCACGTCATCCACGAAGACGACGCCTAAGTACAA

GGTAAAGAACGGCGATAATCTCACCAAAATTGCCAAGAAACACAATACGACAGTGGCTACTTTAT

TAAAACTCAATCCAGGCATTAAAGATCCGAACATGATTCGTGTCGGTCAAACATTAAATGTGACA

GGGAGCGGCGGTAAGACACACAAAGTAAAGAGTGGTGATACCTTGAGCAAGATTGCCGTGGATAA

TAAGACTACAGTTAGTAAGTTGATGAATTTGAATCCTGAGATCACAAATCCTAATCATATCAAAG

TAGGGCAGACCATTCGCTTAAGCCGGAAGCTCCGTCGGCTTAAACGTAAGATCGCTCACAAGGTA

AAAAAATAT;
```

or
(vi) a polynucleotide comprising a DNA sequence having sequence identity of 80% or higher with the polynucleotide comprising a DNA sequence as defined in (iv) or (v), and encoding a polypeptide capable of permeating a lipopolysaccharide-containing outer membrane of Gram-negative bacteria.

(3) An antibacterial protein comprising an endolysin and the polypeptide according to (1), wherein the polypeptide according to (1) is located at the N-terminus or C-terminus of the endolysin.
(4) A polynucleotide encoding the antibacterial protein according to (3).
(5) An antibacterial agent or disinfectant against Gram-negative bacteria, comprising the antibacterial protein according to (3).
(6) The antibacterial agent or disinfectant according to (5), wherein the Gram-negative bacteria are multidrug-resistant bacteria or *Pseudomonas aeruginosa.*
(7) A pharmaceutical composition comprising the antibacterial protein according to (3).
(8) The pharmaceutical composition according to (7), wherein a disease caused by the *Pseudomonas aeruginosa* is one or more diseases selected from the group consisting of keratitis, dacryocystitis, endophthalmitis, cellulitis-induced eye infections, sepsis, pneumonia, multidrug-resistant Gram-negative bacterial infections, opportunistic infections, and other *Pseudomonas aeruginosa*-induced diseases.
(9) An antibacterial composition or disinfectant composition comprising:

the antibacterial protein according to (3); and
an outer membrane permeabilizer,

wherein the outer membrane permeabilizer is ethylenediaminetetraacetic acid (EDTA) or a salt thereof.

(10) The antibacterial composition or disinfectant composition according to (9), wherein the Gram-negative bacteria are multidrug-resistant bacteria or *Pseudomonas aeruginosa.*

(11) A method for preventing a disease caused by a Gram-negative bacterial infection or for killing Gram-negative bacteria, the method comprising administering a pharmaceutical composition containing the antibacterial protein according to (3) to a human.

(12) A method for preventing a disease caused by a Gram-negative bacterial infection or for killing Gram-negative bacteria, the method comprising administering a pharmaceutical composition containing the antibacterial protein according to (3) to an animal.

Advantageous Effects of Invention

The present invention can provide an antibacterial agent, a disinfectant, an antibacterial composition, and a disinfectant composition having efficacy against Gram-negative bacteria including *Pseudomonas aeruginosa,* particularly multidrug-resistant Gram-negative bacteria.

Brief Description of Drawings

Fig. 1 shows the measurement results of the viable bacterial count.
Fig. 2 shows the measurement results of the viable bacterial count.
Fig. 3 shows the morphological observation on bactericidal action against *Pseudomonas aeruginosa* strain PAO1.
Fig. 4 shows the turbidity of *Pseudomonas aeruginosa* strain PAO1 and comparison of morphological observations
Fig. 5 shows a growth inhibitory effect against *Pseudomonas aeruginosa* strain PAO1.
Fig. 6 shows a bactericidal action against *Pseudomonas aeruginosa.*
Fig. 7 shows a bactericidal action against various bacteria.
Fig. 8 shows the results of a 2-day observation of survival in a mouse sepsis model.
Fig. 9 shows the method and results of a treatment experiment on *Pseudomonas* aeruginosa-infected mice.
Fig. 10 shows the results from efficacy study in a mouse keratitis model.

Description of Embodiments

As used in this specification, the singular forms (e.g., a, an, and the) are intended to include the plural forms, unless otherwise explicitly stated or clearly contradicted by the context.

As used in this specification, the terms "include," "contain," and variations express concepts that encompass "comprise," "consist essentially of," and "consist of."

The antibacterial protein of the present invention comprises an outer membrane-permeable polypeptide and an endolysin. The outer membrane-permeable polypeptide and the endolysin may be linked directly or via a suitable linker. The linker may be a polypeptide comprising one amino acid or any two or more amino acids.

As used in this specification, the term "amino acid" encompasses 20 amino acids that constitute proteins (Gly, Ala, Met, Ser, Thr, Cys, Met, Asp, Asn, Glu, Gln, Leu, Ile, Val, His, Lys, Arg, Phe, Tyr, and Trp).

As used in this specification, the expression "deletion of an amino acid residue" or similar expressions refer to the deletion of any amino acid residue selected from the amino acid sequence of the outer membrane-permeable polypeptide or endolysin. The expression "insertion of an amino acid residue" or similar expressions refer to the insertion of one to several amino acid residues into a site other than the N-terminus or C-terminus of the amino acid sequence of the outer membrane-permeable polypeptide or endolysin. The expression "addition of an amino acid residue" or similar expressions refer to the addition of one to several amino acid residues to the N-terminus or C-terminus of the amino acid sequence of the outer membrane-permeable polypeptide or endolysin. Amino acid substitutions include, for example, conservative amino acid substitutions. The term "conservative amino acid substitutions" refers to substitutions between amino acid residues that have similar properties, such as polarity, electrical properties, and structural characteristics. Examples include substitutions among residues of hydrophobic amino acids, polar amino acids, acidic amino acids, basic amino acids, amino acids with branched side chains, and aromatic amino acids. Examples of hydrophobic (nonpolar) amino acids include glycine, alanine, valine, leucine, isoleucine, and proline. Examples of polar amino acids include serine, threonine, cysteine, methionine, asparagine, and glutamine. Examples of acidic amino acids include aspartic acid and glutamic acid. Examples of basic amino acids include lysine, arginine, and histidine. Examples of amino acids with branched side chains include valine, isoleucine and leucine. Examples of aromatic amino acids include phenylalanine, tyrosine, tryptophan, and histidine. Examples of preferred conservative amino acid substitutions include amino acid substitutions selected from the following: a substitution among valine, leucine, and isoleucine; a substitution between phenylalanine and tyrosine; a substitution between lysine and arginine; a substitution between alanine and valine; and a substitution between asparagine and glutamine.

The deletion, substitution, insertion, and/or addition of an amino acid residue can be achieved by modifying a gene encoding an antibacterial protein, an outer membrane-permeable polypeptide, or an endolysin by publicly known techniques in the relevant field. Mutations can be introduced into genes by publicly known methods, such as the Kunkel method, the Gapped duplex method, or other methods equivalent to the above methods. For example, mutations can be introduced using a mutation introduction kit that utilizes site-directed mutagenesis (e.g., Mutant-K available from Takara Bio Inc. or Mutant-G available from Takara Bio Inc.) or LA PCR in vitro Mutagenesis series kit available from Takara Bio Inc.

The number of amino acid residues in the linker is preferably 1, 2, 3, 4, 5, 6, 7, 8, 9, or 10, more preferably 1, 2, 3, 4, 5, or 6, and even more preferably 1, 2, 3, or 4. Preferably, the outer membrane-permeable polypeptide and the endolysin are directly linked. Preferably, the outer membrane-permeable polypeptide is located at the C-terminus of the endolysin.

(1) Outer membrane-permeable polypeptide and polynucleotide encoding the polypeptide

The outer membrane-permeable polypeptide is a polypeptide as defined in one of (i) to (iii) below:

(i) a polypeptide comprising the amino acid sequence represented by SEQ ID NO: 1;
(ii) a polypeptide comprising an amino acid sequence in which one or more amino acid residues are deleted, substituted, inserted, and/or added in the amino acid sequence represented by SEQ ID NO: 1, and capable of permeating a lipopolysaccharide-containing outer membrane of Gram-negative bacteria; or
(iii) a polypeptide comprising an amino acid sequence having sequence identity of 80% or higher with the amino acid sequence represented by SEQ ID NO: 1, and capable of permeating a lipopolysaccharide-containing outer membrane of Gram-negative bacteria.

The polypeptide represented by SEQ ID NO: 1 comprises the D domain and S domain shown below, which are linked together, with the D domain positioned at the N-terminus of the polypeptide and the S domain positioned at the C-terminus of the polypeptide (DS). The polypeptide (SD), which comprises the S domain at the N-terminus and the D domain at the C-terminus, has a significantly weak action of enhancing the antibacterial action of an endolysin against Gram-negative bacteria, compared to the DS. The amino acid sequence (SEQ ID NO: 3) of the D domain is as follows:

NSGTPKNVSRGTSSTKTTPKYKVKNGDNLTKIAKKHNTTVATLLKLNPGIKDPNMIRVGQTLNVT

GSGGKTHKVKSGDTLSKIAVDNKTTVSKLMNLNPEITNPNHIKVGQTIRLS.

The amino acid sequence (SEQ ID NO: 4) of the S domain is as follows: RKLRRLKRKIAHKVKKY.

A particularly preferred outer membrane-permeable polypeptide is one represented by SEQ ID NO: 1.

As long as the outer membrane-permeable polypeptide is capable of permeating a lipopolysaccharide-containing outer membrane of Gram-negative bacteria, one or more (e.g., 9, 8, 7, 6, 5, 4, 3, or 2) amino acid residues may be deleted, substituted, inserted, and/or added in the polypeptide represented by SEQ ID NO: 1.

The outer membrane-permeable polypeptide may be one having sequence identity of 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher with the amino acid sequence represented by SEQ ID NO: 1. The value of sequence identity refers to the value calculated using software for computing identity among multiple amino acid sequences (e.g., FASTA, DANASYS, and BLAST) with the default settings.

In one preferred embodiment, the polynucleotide of the present invention is a polynucleotide encoding the outer membrane-permeable polypeptide.

A preferred polynucleotide of the present invention encoding the outer membrane-permeable polypeptide is as defined below:

(iv) a polynucleotide comprising a DNA sequence encoding the polypeptide represented by SEQ ID NO: 1;
(v) a polynucleotide comprising the DNA sequence represented by SEQ ID NO: 2; or
(vi) a polynucleotide comprising a DNA sequence having sequence identity of 80% or higher with the polynucleotide comprising a DNA sequence as defined in (iv) or (v), and encoding a polypeptide capable of permeating a lipopolysaccharide-containing outer membrane of Gram-negative bacteria.

The polynucleotide encoding the outer membrane-permeable polypeptide may be one having sequence identity of 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher with the polynucleotide comprising a DNA sequence encoding the amino acid sequence represented by SEQ ID NO: 1 or the polynucleotide represented by SEQ ID NO: 2. The value of sequence identity refers to the value calculated using software for computing identity among multiple amino acid sequences (e.g., FASTA, DANASYS, and BLAST) with the default settings.

(2) Endolysin

Endolysins are proteins having peptidoglycan degrading activity. As used in this specification, the term "peptidoglycan degrading activity" refers to the activity of binding to or recognizing peptidoglycan in the cell walls of Gram-negative bacteria, including *Pseudomonas aeruginosa,* and cleaving the peptidoglycan to specifically cause bacteriolysis.

The endolysin may be publicly known endolysin that is widely used. Examples include endolysins derived from phages of Gram-negative bacteria. Specific examples of endolysins include, but are not limited to, a polypeptide called "ply_pitti26" isolated from the lytic phage pitti26 and used for symptoms of staphylococcal infections (JP2010-536354A); S. *aureus* phage φ2638a endolysin (JP6261086B); phage phi MR11-derived endolysin (J Infect Dis. 196(8):1237-47, (2007)); *Pseudomonas aeruginosa* phage PAJU2-derived endolysin (Virus Research. 139(1), 131-134, 2009); phage S25-3-derived endolysin (Viruses, 11(9):769, 2019); two types of endolysins isolated from bacteriophage S6 (JP2021-112152A); the endolysins disclosed in NPL 1 to NPL 3; *Bacillus amyloliquefaciens* phage-derived endolysin (FEBS Letter. 500 (1-2), 56-59, 2001); and Artilysins (mBio. 2014 Jul 1;5(4):e01379-14. doi: 10.1128/mBio.01379-14).

The endolysin contained in the antibacterial protein of the present invention may be a modified protein of the endolysin as long as it has the eptidoglycan degrading activity described above. Modified proteins include proteins comprising an amino acid sequence in which one or more (e.g., 9, 8, 7, 6, 5, 4, 3, or 2) amino acid residues are deleted, substituted, inserted, and/or added to an amino acid sequence of a publicly known endolysin, and having the peptidoglycan degrading activity; and proteins comprising an amino acid sequence having a sequence identity of 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, with an amino acid sequence of publicly known endolysin, and having the peptidoglycan degrading activity. The value of sequence identity refers to the value calculated using software for computing identity among multiple amino acid sequences (e.g., FASTA, DANASYS, and BLAST) with the default settings.

(3) Antibacterial protein and production method

The antibacterial protein of the present invention comprises an outer membrane-permeable polypeptide and an endolysin. A preferred antibacterial protein comprises an endolysin located at its N-terminus and an outer membrane-permeable polypeptide located at its C-terminus. The antibacterial protein can be fused with tags such as Strep, His, FLAG, Xpress, Avi, calmodulin, polyglutamate, HA, Myc, Nus, S, X, SBP, Sof, V5, CBP, GST, MBP, GFP, and thioredoxin tags, as well as combinations of these tags. A 6xHis tag is preferred.

The antibacterial protein, antibacterial agent, disinfectant, antibacterial composition, and disinfectant composition of the present invention have efficacy against Gram-negative bacteria, particularly multidrug-resistant Gram-negative bacteria. Examples of Gram-negative bacteria include *Pseudomonas aeruginosa, Escherichia coli,* enterohemorrhagic *Escherichia coli* (EHEC), Enterobacteriaceae, *Vibrio, Campylobacter, Salmonella, Legionella,* cholera, *Haemophilus influenzae, Klebsiella, Enterobacter, Serratia,* pertussis, *Yersinia, Pseudomonas,* and *Helicobacter pylori.* More specific examples include *Escherichia coli, Klebsiella pneumoniae, Pseudomonas aeruginosa, Enterobacter* spp., *Serratia* marcescens, *Acinetobacter* spp., *Proteus* spp., *Klebsiella* oxytoca, *Bacteroides* spp. including *Bacteroides fragilis, Citrobacter* spp., *Haemophilus influenzae, Burkholderia cepacia, Stenotrophomonas maltophilia, Chryseobacterium* spp., and *Salmonella enterica.*

Examples of multidrug-resistant Gram-negative bacteria include multidrug-resistant *Acinetobacter* (MDRA), multidrug-resistant *Pseudomonas aeruginosa* (MDRP), and carbapenem-resistant *Enterobacteriaceae* bacteria.

The antibacterial protein of the present invention can be produced by genetic engineering methods by using a polynucleotide that contains a gene encoding the antibacterial protein. For example, the antibacterial protein can be produced by preparing RNA by in vitro transcription from a recombinant vector having a polynucleotide that contains a gene encoding the antibacterial protein, and then using the RNA as a template for in vitro translation. Alternatively, the antibacterial protein can be produced by operably linking and incorporating a polynucleotide that contains a gene encoding the antibacterial protein to a suitable vector, introducing the vector into host cells to produce transformed cells, and then expressing the desired antibacterial protein from the transformed cells. As used in this specification, the term "expression" includes any step involved in producing a protein, including transcription, post-transcriptional modification, translation, post-translational modification, and secretion.

The vector can be appropriately selected and used from those suitable for the host cells into which the vector is introduced. The vector also contains a polynucleotide that contains a gene encoding the antimicrobial protein of the present invention operably linked to an appropriate promoter, and preferably contains a transcription termination signal, i.e., a terminator region, downstream of the polynucleotide. Further, the vector may contain a selectable marker gene (such as a drug resistance gene or a gene that complements an auxotrophic mutation) used for selecting a transformant. The vector may also contain, for example, a sequence encoding a tag sequence useful for separating and purifying the expressed protein. The vector may also be one that is incorporated into the genome of the host cells.

The vector can be introduced into the host cells by a publicly known transformation method, such as the competent cell method, the protoplast method, or the calcium phosphate coprecipitation method.

The host cells into which the vector are introduced for expressing a recombinant protein may be any cells capable of expressing the vector, including commonly used and publicly known microorganisms, such as bacteria, yeast, fungi, and mammalian cells. Examples of bacteria include Gram-positive bacteria, including *Bacillus* and *Streptomyces.* Recombinant cells can be cultured by a publicly known method suitable for the host cells.

The expressed protein can be purified by disrupting, with ultrasound, glass beads, or the like, the bacterial cells harvested from the culture supernatant of the host cells by centrifugation or the like; removing solids, such as cell debris by centrifugation or the like to prepare a crude enzyme solution; and then purifying the crude enzyme solution, for example, by one or a combination of the following publicly known methods for purifying proteins or peptides: ammonium sulfate precipitation, precipitation with organic solvents (ethanol, methanol, acetone, etc.), ion exchange chromatography, isoelectric focusing chromatography, gel filtration chromatography, hydrophobic chromatography, adsorption column chromatography, affinity chromatography using a substrate or antibody, reverse phase column chromatography, chromatography such as HPLC, and filtration processes, such as microfiltration, ultrafiltration, and reverse osmosis.

The antimicrobial protein of the present invention can also be produced by chemical synthesis based on its amino acid sequence. In the case of chemical synthesis, the antibacterial protein can be produced by known chemical synthesis methods, such as the Fmoc method (fluorenylmethoxycarbonyl method) or the tBoc method (t-butoxycarbonyl method).

The present invention also provides the antibacterial protein, antibacterial agent or disinfectant, pharmaceutical composition, and antibacterial composition or disinfectant composition of the present invention for use as human pharmaceutical products. The antibacterial agent, disinfectant, antibacterial composition, and disinfectant composition of the present invention may contain a "pharmaceutically acceptable carrier." Examples of those acceptable for use in mammals, including humans, include any adjuvants, carriers, excipients, glidants, sweeteners, diluents, preservatives, dyes/colorants, flavoring agents, surfactants, wetting agents, dispersing agents, suspending agents, stabilizers, isotonic agents, solvents, surfactants, and emulsifiers that are approved by the Japanese Pharmacopoeia, the U.S. Food and Drug Administration, etc. Example pharmaceutically acceptable carriers include, but are not limited to sugars, such as lactose, glucose, and sacrose; starches, such as corn starch and potato starch; cellulose and derivatives, such as sodium carboxymethylcellulose, ethyl cellulose, and cellulose acetate; tragacanth; malt; gelatin; talc; cocoa butter; waxes; tallow and vegetable fats; paraffin; silicone; bentonite; silicic acid; zinc oxide; oils, such as peanut oil, cottonseed oil, safflower oil, sesame oil, olive oil, corn oil, and soybean oil; glycols, such as propylene glycol; polyols, such as glycerol, sorbitol, mannitol, and polyethylene glycol; esters, such as ethyl oleate and ethyl laurate; agar; buffers, such as magnesium hydroxide and aluminum hydroxide; alginic acid, pyrogen-free water; water for injection; saline, preferably physiological saline; Ringer's solution; ethyl alcohol, and phosphate buffer.

The antibacterial agent, disinfectant, antibacterial composition, and disinfectant composition of the present invention are formulated for parenteral administration, for example, intravascular (intravenous or intraarterial) administration. In a preferred embodiment, the antibacterial agent and the antibacterial composition contemplated in this specification may be injected intravenously into a mammalian subject, such as a human, in a single dose.

Preferred embodiments of the antibacterial agent, disinfectant, antibacterial composition, and disinfectant composition of the present invention include the use thereof for diseases, such as pneumonia, bacteremia, sepsis, tetanus, *Salmonella* infection, meningitis, scarlet fever, scrub typhus, sexually transmitted bacterial diseases, surgical site infections, necrotizing fasciitis, *E. coli* infection, bacterial skin diseases, *Campylobacter* infection, cholera, gastritis, peptic ulcer, diarrhea, dysentery, food poisoning, typhoid fever, Legionnaires' disease, opportunistic infections, urinary tract infections, and whooping cough; or the use thereof for disinfecting hands, medical instruments, or medical materials. For example, the above products can be used for the purpose of disinfecting medical equipment and peripheral equipment that may be contaminated with *Pseudomonas aeruginosa* in the eyes, urinary tract, or respiratory system.

In another preferred embodiment, the present invention can be used for diseases in the field of ophthalmology. The present invention can be used in the form of eye drops, eye ointments, etc. for keratitis, corneal ulcers, blepharitis, conjunctivitis, stye, tarsadenitis, and dacryocystitis; for prevention of bacterial infections before and after ophthalmic surgery including cataract surgery; and for contact lens preservatives and the like.

The antibacterial protein of the present invention can also be used for animals (for veterinary use). For veterinary use, the antibacterial agent, disinfectant, antibacterial composition, and disinfectant composition of the present invention are typically administered in the form of acceptable formulations according to normal veterinary practice, and a veterinarian can decide the dosing regimen and route of administration most suitable for a particular animal. Also in the case of veterinary use, the above products may contain any of the pharmaceutically acceptable carriers described above, may be administered via any of the routes described above, and may be used for disinfection for the purpose described above or for prevention or treatment of ophthalmic diseases.

As used in this specification, the term "animal" includes mammals, non-mammals, birds, and the like. Preferably, the animals in the present invention include the livestock, companion animals, laboratory animals, and captive animals. More specifically, the animals in the present invention include, but are not limited to, cattle, horses, donkeys, camels, alpacas, pigs, goats, sheep, dogs, cats, chinchillas, rabbits, minks, ferrets, guinea pigs, mice, rats, hedgehogs, weasels, kangaroos, chimpanzees, gorillas, orangutans, crab-eating macaques, baboons, chickens, pigeons, turkeys, pheasants, ostriches, guinea fowl, ducks, and wild ducks.

Furthermore, in the case of veterinary use, the antibacterial protein of the present invention can also be used for the purpose of preventing or treating typical infectious diseases in animals. Such typical infectious diseases in animals include those described in Journal of the Japan Veterinary Medical Association, 29, 429-433 (1976). More specifically, examples of infectious diseases in animals that are targets of the antibacterial protein of the present invention include, but are not limited to acute or chronic mastitis, genital infections, sepsis, and sole ulcers (hoof disease) in cattle; endometritis, genital infections, and pulmonary suppuration in horses; bronchopneumonia, fibrous pleurisy, and hemorrhagic enteritis in pigs; otitis externa, genital infections, urethritis, endocarditis, pneumonia, sepsis, and eye inflammation in dogs; and otitis externa, genital infections, urethritis, endocarditis, pneumonia, sepsis, and eye inflammation in cats.

In another embodiment, the present invention provides the use of the antibacterial protein of the present invention in the production of human or veterinary pharmaceutical products for prevention or treatment of a medical condition caused by a Gram-negative bacterial infection in humans or animals.

Examples

The present invention will be described in more detail with reference to the following examples.

**Example 1**

A protein was created by fusing the endolysin (YP_002284408; 138aa; SEQ ID NO: 5) of *Pseudomonas aeruginosa* phage PAJU2, a partial sequence (AAK40280, 143-258aa; SEQ ID NO: 6) of endolysin (*Bacillus amyloliquefaciens* phage Morita2001), and SMAP17 (RKLRRLKRKIAHKVKKY), a peptide generated by introducing mutations into a partial sequence (AAA85470, 1-17aa; SEQ ID NO: 7) of a sheep antibacterial peptide SMAP29.

The following base sequences were used.

[Table 1]

| ORF27 (27) 144aa SEQ ID NO: 5 | ATGCGTACATCCCAACGAGGCATCGACCTCATCAAATCCTTC GAGGGCCTGCGCCTGTCCGCTTACCAGGACTCGGTGGGT GTCTGGACCATAGGTTACGGCACCACTCGGGGCGTCACCC GCTACATGACGATCACCGTCGAGCAGGCCGAGCGGATGCT GTCGAACGACATTCAGCGCTTCGAGCCAGAGCTAGACAGG CTGGCGAAGGTGCCACTGAACCAGAACCAGTGGGATGCCC TGATGAGCTTCGTGTACAACCTGGGCGCGGCCAATCTGGC GTCGTCCACGCTGCTCAAGCTGCTGAACAAGGGTGACTAC CAGGGAGCAGCGGACCAGTTCCCGCGCTGGGTGAATGCG GGCGGTAAGCGCTTGGATGGTCTGGTTAAGCGTCGAGCAG CCGAGCGTGCGCTGTTCCTGGAGCCACTATCG |

(continued)

| Morita2001 (D) 116aa SEQ ID NO: 6 | AATTCTGGCACGCCTAAGAACGTATCGCGCGGCACGTCATC CACGAAGACGACGCCTAAGTACAAGGTAAAGAACGGCGATA ATCTCACCAAAATTGCCAAGAAACACAATACGACAGTGGCTA CTTTATTAAAACTCAATCCAGGCATTAAAGATCCGAACATGAT TCGTGTCGGTCAAACATTAAATGTGACAGGGAGCGGCGGTA AGACACACAAAGTAAAGAGTGGTGATACCTTGAGCAAGATT GCCGTGGATAATAAGACTACAGTTAGTAAGTTGATGAATTTG AATCCTGAGATCACAAATCCTAATCATATCAAAGTAGGGCAG ACCATTCGCTTAAGC |
| SMAP17 (S) 17aa SEQ ID NO: 7 | CGGAAGCTCCGTCGGCTTAAACGTAAGATCGCTCACAAGGT AAAAAAATAT |

The fusion genes DS and SD were cloned, and 27 was also cloned. Cloning was performed to allow expression of a protein fused with 27 and DS or SD. A restriction enzyme site was present at each terminus.

Cloned genes

pBluescript SK(-) SalI-DS-XbaI
pBluescript SK(-) SalI-SD-XbaI
pBluescript SK(-) SacI-DS-XhoI
pBluescript SK(-) SacI-SD-XhoI
pColdII XhoI-27-EcoRI

SalI-DS-XbaI, SalI-SD-XbaI, SacI-DS-XhoI, and SacI-SD-XhoI were introduced into pColdII XhoI-27-EcoRI to produce the following fusion protein expression plasmids.

Table 2

| Plasmid | Expressed protein |
| --- | --- |
| pColdII SacI-DS-XhoI-27-EcoRI | DSx27 |
| pColdII SacI-OS-XhoI-27-EcoRI | SDx27 |
| pColdII XhoI-27-EcoRI- SalI-SD-XbaI | 27xSD |
| pColdII XhoI-27-EcoRI- SalI-DS-XbaI | 27xDS |

**Protein expression**

The above plasmids were introduced into BL21 strain.
The protein expression protocols are as follows.

Expression method

1. A single colony was picked from LB medium containing 100 $\mu$g/ml ampicillin, inoculated into LB medium containing 100 $\mu$g/ml ampicillin, and cultured overnight with shaking at 37°C.
2. The overnight cultured bacteria were added to a culture medium (LB + 100 $\mu$g/ml ampicillin). When the OD600 reached 1.0, the culture medium was cooled on ice and left standing for 30 minutes.

   * Since it takes time to reach an OD of 1.0, in the case of a 1 L culture medium, approximately 40 ml of overnight culture was used.

* In Takara's protocol, the OD 600 is 0.4 to 0.5. However, the results from investigation of this protein expression showed that the expression level was the highest at an OD of 1.0.

3. IPTG was added to a concentration of 1.0 mM, and the mixture was cultured with shaking at 15°C for 24 hours.

Purification method

1. After incubation, the cells were centrifuged (10,000 × g, 10 minutes, 4°C) to obtain a pellet.
2. 25 mL of 100 mM phosphate buffer (300 mM NaCl, pH of 7.8) was added to the bacterial pellet, and the mixture was homogenized on ice for 5 minutes (5 seconds ON/5 seconds OFF) using a Q700 ultrasonic homogenizer (WakenBtech Co., LTD., Kyoto, Japan) (25 mL of phosphate buffer for 250 mL of culture medium).
3. After ultrasonic treatment, the mixture was centrifuged (8,000 × g, 20 minutes, 4°C) and the supernatant was collected. HIGH Density COBALT (ProteNova Co., Ltd., Kagawa, Japan) equilibrated with 100 mM phosphate buffer (300 mM NaCl, pH of 7.8) was added to the resulting supernatant, and the mixture was allowed to bind overnight at 4°C with rotation.
4. The protein-bound HIGH Density COBALT was allowed to fall by gravity, and the excess turbid liquid (supernatant) was discarded.
5. 25 ml of 100 mM phosphate buffer (300 mM NaCl, pH of 7.8) was added, and the HIGH Density COBALT was washed for 10 minutes at 4°C with rotation.
6. Then, the HIGH Density COBALT bound to the protein was allowed to fall by gravity, and the excess phosphate buffer (supernatant) was discarded.
7. Steps 5 and 6 were repeated twice.
8. 500 μl of phosphate buffer containing imidazole at a final concentration of 500 mM was added, and the eluted protein was collected.

After dialysis against PBS, the protein concentration was measured, and the enzyme activity was measured according to the following procedure.

**Preparation of bacteria**

0.25 ml of overnight cultured *Pseudomonas aeruginosa* strain PAO1 was added to 7 ml of LB broth (18 mm test tube) and cultured with shaking at 37°C.

↓ 2 to 3 hours

The bacterial cells were harvested when the $OD_{660}$ reached around 0.6, as monitored using a Taitec Digital colorimeter Easy operation OD Monitor Miniphoto 518R.

↓

The cells were washed with PBS once (the collected culture was centrifuged, the supernatant was discarded, the pellet was suspended in PBS and centrifuged, and PBS was added).

↓

Using a Taitec Digital colorimeter Easy operation OD Monitor Miniphoto 518R, the bacterial turbidity was adjusted with PBS to attain an $OD_{660}$ of around 0.2.

**Protocol for measuring viable bacterial count**

The components described below were mixed in the wells of a 96-well plate and cultured with shaking at 37°C for 30 minutes. 10 μL of the stock solution and its serially diluted samples were dropped into square Petri dishes, followed by culturing.

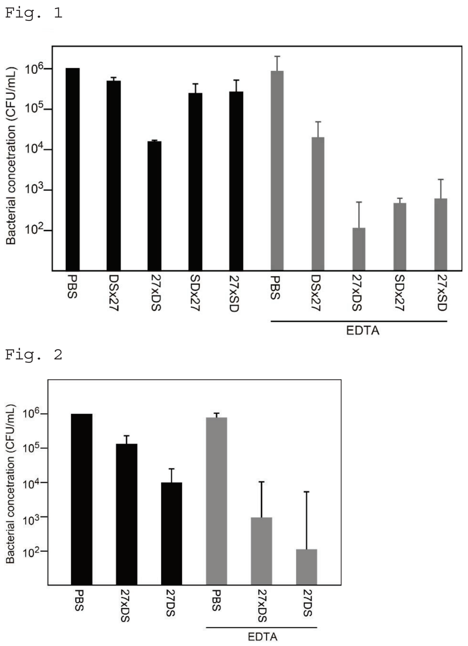

Examination of bactericidal effect of protein alone (Fig. 1)

Protein treatment: bacterial suspension 50 μL + protein 25 μL (final 0.1 mg/ml) + PBS 25 μL
Control: bacterial suspension 50 μL + PBS 50 μL

Examination of bactericidal effect of protein when EDTA is added (Fig. 1)

Protein treatment: bacterial suspension 50 μL + protein 25 μL (final 0.025 mg/ml) + EDTA 25 μL (final 0.5 mM)
Control: bacterial suspension 50 μL + PBS 25 μL + EDTA 25 μL (final 0.5 mM)

Based on these results, the most active, 27xDS, was selected.
Next, the effect of the presence or absence of a linker between 27 and DS in 27xDS was examined.

[Table 3]

| Plasmid | Expressed protein |
|---|---|
| pColdII XhoI-27-EcoRI- SalI-DS-XbaI | 27xDS |
| pColdIV 27DS | 27DS |

The same method as described above was used.
Fig. 2 shows the results.
Based on the above, 27DS was selected as a candidate molecule.

Expressed protein sequences of 27xDS and 27DS

>27xDS (SEQ ID NO: 8)

HMELGTLEMRTSQRGIDLIKSFEGLRLSAYQDSVGVWTIGYGTTRGVTRYMTITVEQAERMLSND
IQRFEPELDRLAKVPLNQNQWDALMSFVYNLGAANLASSTLLKLLNKGDYQGAADQFPRWVNAGG
KRLDGLVKRRAAERALFLEPLSKLVDNSGTPKNVSRGTSSTKTTPKYKVKNGDNLTKIAKKHNTT
VATLLKLNPGIKDPNMIRVGQTLNVTGSGGKTHKVKSGDTLSKIAVDNKTTVSKLMNLNPEITNP
NHIKVGQTIRLSRKLRRLKRKIAHKVKKYSRHHHHHH (54 + 62 = 116)

>27DS (SEQ ID NO: 9)

HMMRTSQRGIDLIKSFEGLRLSAYQDSVGVWTIGYGTTRGVTRYMTITVEQAERMLSNDIQRFEP
ELDRLAKVPLNQNQWDALMSFVYNLGAANLASSTLLKLLNKGDYQGAADQFPRWVNAGGKRLDGL
VKRRAAERALFLEPLSNSGTPKNVSRGTSSTKTTPKYKVKNGDNLTKIAKKHNTTVATLLKLNPG
IKDPNMIRVGQTLNVTGSGGKTHKVKSGDTLSKIAVDNKTTVSKLMNLNPEITNPNHIKVGQTIR
LSRKLRRLKRKIAHKVKKYHHHHHH

Gene sequence of 27DS
>27DS (SEQ ID NO: 10)

ATGATGCGTACATCCCAACGAGGCATCGACCTCATCAAATCCTTCGAGGGCCTGCGCCTGTCCGC

TTACCAGGACTCGGTGGGTGTCTGGACCATAGGTTACGGCACCACTCGGGGCGTCACCCGCTACA

TGACGATCACCGTCGAGCAGGCCGAGCGGATGCTGTCGAACGACATTCAGCGCTTCGAGCCAGAG

CTAGACAGGCTGGCGAAGGTGCCACTGAACCAGAACCAGTGGGATGCCCTGATGAGCTTCGTGTA

CAACCTGGGCGCGGCCAATCTGGCGTCGTCCACGCTGCTCAAGCTGCTGAACAAGGGTGACTACC

AGGGAGCAGCGGACCAGTTCCCGCGCTGGGTGAATGCGGGCGGTAAGCGCTTGGATGGTCTGGTT

AAGCGTCGAGCAGCCGAGCGTGCGCTGTTCCTGGAGCCACTATCGAATTCTGGCACGCCTAAGAA

CGTATCGCGCGGCACGTCATCCACGAAGACGACGCCTAAGTACAAGGTAAAGAACGGCGATAATC

TCACCAAAATTGCCAAGAAACACAATACGACAGTGGCTACTTTATTAAAACTCAATCCAGGCATT

AAAGATCCGAACATGATTCGTGTCGGTCAAACATTAAATGTGACAGGGAGCGGCGGTAAGACACA

CAAAGTAAAGAGTGGTGATACCTTGAGCAAGATTGCCGTGGATAATAAGACTACAGTTAGTAAGT

TGATGAATTTGAATCCTGAGATCACAAATCCTAATCATATCAAAGTAGGGCAGACCATTCGCTTA

AGCCGGAAGCTCCGTCGGCTTAAACGTAAGATCGCTCACAAGGTAAAAAAATATCATCATCATCA

TCATCATTAG

**Example 2:** Examination of in vitro bactericidal action of 27DS **Preparation of bacteria**

0.25 ml of overnight cultured *Pseudomonas aeruginosa* strain PAO1 was added to 7 ml of LB broth (18 mm test tube) and cultured with shaking at 37°C.
↓ 2 to 3 hours
The bacterial cells were harvested when the $OD_{660}$ reached around 0.6, as monitored using a Taitec Digital colorimeter Easy operation OD Monitor Miniphoto 518R.
↓
The cells were washed with PBS once (the collected culture was centrifuged, the supernatant was discarded, the pellet was suspended in PBS and centrifuged, and PBS was added).
↓
Using a 96-well spectrophotometer (Multiskan FC available from Thermo Scientific), the bacterial turbidity was adjusted with PBS to attain an $OD_{595}$ of 0.5 to 1.0.

2.1. Morphological observation of bactericidal action against *Pseudomonas aeruginosa* strain PAO1

The components described below were mixed in each well of a 96-well plate.

Examination of bactericidal effect of 27DS alone

27DS treatment: bacterial suspension 50 μL + 27DS 25 μL (final 0.1 mg/ml) + PBS 25 μL
Control: bacterial suspension 50 μL + PBS 50 μL

Examination of bactericidal effect of 27DS when EDTA is added

27DS treatment: bacterial suspension 50 μL + 27DS 25 μL (final 0.025 mg/ml) + EDTA 25 μL (final 0.5 mM)
Control: bacterial suspension 50 μL + PBS 25 μL + EDTA 25 μL (final 0.5 mM)

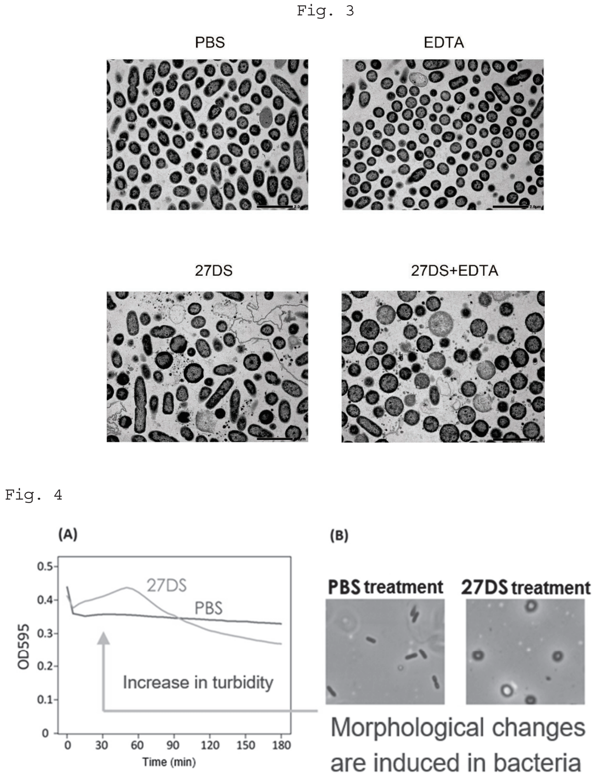

The mixture was cultured with shaking at 37°C for 30 minutes, and glutaraldehyde (2%) was added to end the reaction. After centrifugation, the bacterial cells were coated with 1% agarose and washed with PBS containing 5% sucrose. The bacterial cells were then fixed with 1.5% osmium tetroxide in 0.1 mol/L phosphate buffer containing 5% sucrose for 1 hour at 4°C. After dehydration in graded ethanol solutions, the fixative was replaced with propylene oxide, and the samples were embedded in epoxy resin. Ultrathin sections were prepared, stained with uranyl acetate and lead citrate, and observed under a transmission electron microscope (Fig. 3).

The results showed bacterial swelling due to the 27DS treatment. The results revealed that 27DS has the effect of disrupting the cell walls. The EDTA treatment tended to cause greater morphological changes in the bacteria. EDTA is considered to have the effect of changing the structure of LPS and the effect of improving the bactericidal action of 27DS. The results are consistent with the results of in vitro tests of the bactericidal effect of 27DS.

2.2. In vitro validation results 1: Observation of turbidity and morphology of strain PAO1 in PBS

The following components were mixed in each well of a 96-well plate.

27DS treatment: bacterial suspension 50 μL + 27DS 25 μL (final 0.1 mg/ml)
PBS 25 μL control: bacterial suspension 50 μL + PBS 50 μL

The mixture was cultured with shaking at 37°C for 0 to 180 minutes. After a certain period of time, the turbidity of the samples taken at each time point was measured at 595 nm, and the changes over time were compared with those of the control. While the turbidity of the control remained nearly constant throughout the experimental period, the turbidity of the 27DS-added samples increased from 0 to 60 minutes and then decreased, suggesting inhibition of growth of the strain PAO1. Furthermore, when the bacterial cells were treated as described in 2.1 and observed under a transmission electron microscope, the results suggest that the 27DS treatment causes morphological changes in the bacterial cells (Fig. 4).

2.3. In vitro validation results 2: Effect of 27DS against PAO1 strain during culture

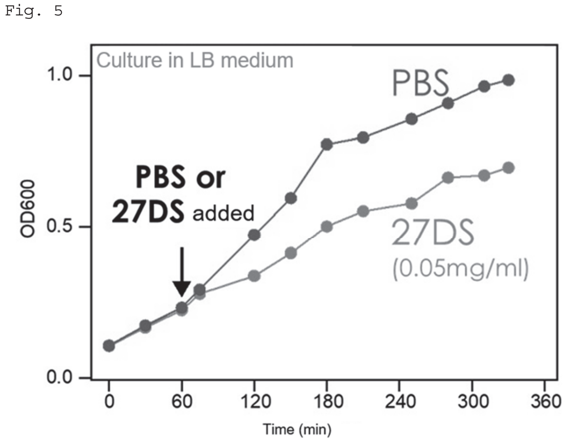

0.15 ml of *Pseudomonas aeruginosa* strain PAO1 was added to 10 ml of LB broth (18 mm test tube) and cultured with shaking at 37°C. Then, 0.1 ml of PBS was added as a control to one culture medium in the logarithmic growth phase (culture for 60 minutes, $OD_{600} = 0.2$), and 0.1 ml of 27DS (5 mg/ml) was added to the other culture medium in the logarithmic growth phase. Then, the culture was continued for 320 minutes from the start of the culture. As a result, after 320 minutes of culture, the $OD_{600}$ was approximately 1.0 in the PBS-added control, whereas the $OD_{600}$ remained at approximately 0.6 in the samples with 27DS added, revealing that PAO1 growth was inhibited (Fig. 5).

2.4. Examination of bactericidal action against various bacteria

**Protocol for measuring viable bacterial count**

The following components were mixed in each well of a 96-well plate.

Examination of bactericidal effect of 27DS alone

27DS treatment: bacterial suspension 50 μL + 27DS 25 μL (final 0.1 mg/ml) + PBS 25 μL
Control: bacterial suspension 50 μL + PBS 50 μL

Examination of bactericidal effect of 27DS when EDTA is added

27DS treatment: bacterial suspension 50 μL + 27DS 25 μL (final 0.1 mg/ml) + EDTA 25 μL (final 0.5 mM)
Control: bacterial suspension 50 μL + PBS 25 μL + EDTA 25 μL (final 0.5 mM). After culturing with shaking at 37°C for 30 minutes, 10 μL of the stock solution and serially diluted samples were dropped into square Petri dishes.

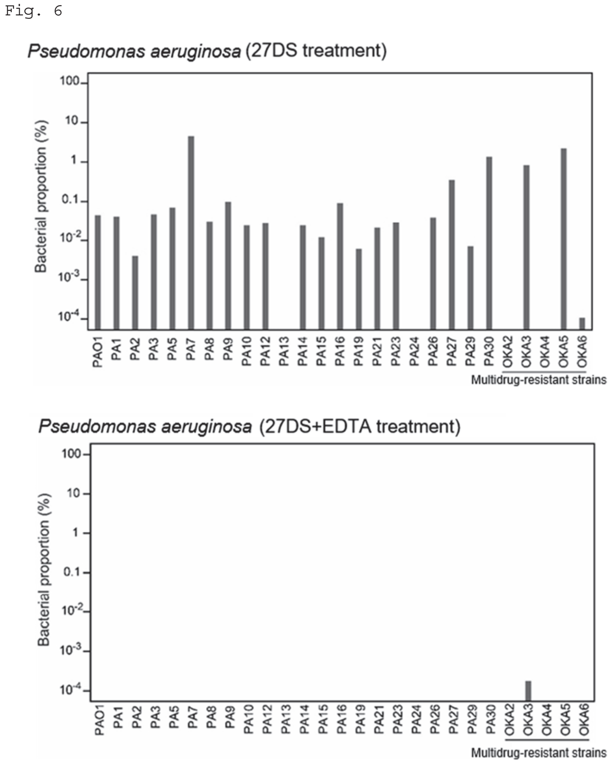

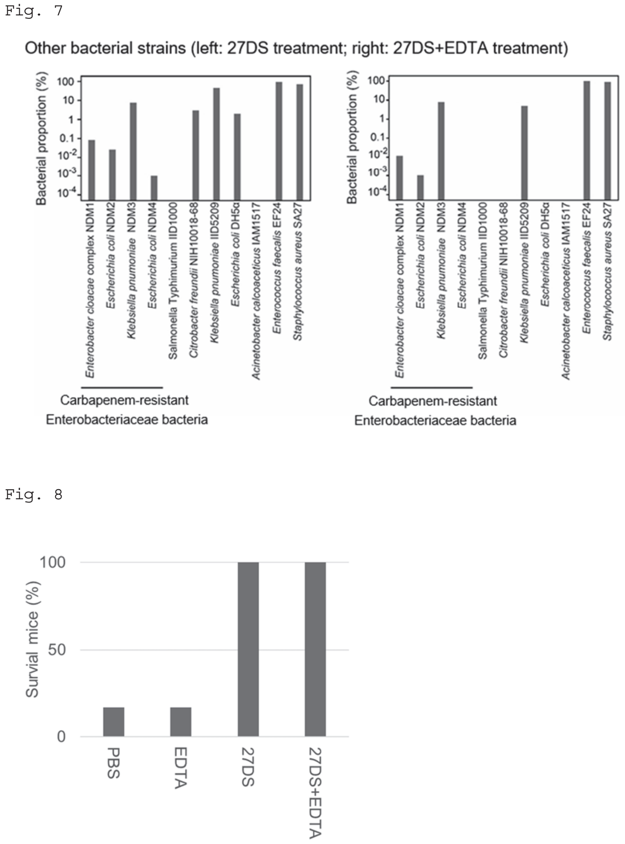

The bacterial count was expressed as a percentage (Fig. 6 and Fig. 7).

Bacterial proportion (%) = Bacterial concentration (treatment group; cfu/mL)/bacterial concentration (PBS group; cfu/mL)

27DS alone can kill *Pseudomonas aeruginosa,* including multidrug-resistant *Pseudomonas aeruginosa.*

27DS + EDTA showed a very high bactericidal effect.

*Enterobacter cloacae* complex NDM1
*Escherichia coli* NDM2
*Escherichia coli* NDM4
*Salmonella typhimurium* IID1000
*Citrobacter freundii* NIH10018-68
*Escherichia coli* DH5α
*Acinetobacter calcoaceticus* IAM1517

The 27DS treatment showed the bactericidal action against the above.

27DS + EDTA treatment showed a high bactericidal action.

27DS treatment and 27DS + EDTA treatment showed weak bactericidal action against *Klebsiella pneumonia* (*Klebsiella pneumoniae* NDM3, *Klebsiella pneumoniae* IID5209).

No bactericidal action was observed against *Enterococcus faecalis* EF24 or *Staphylococcus aureus* SA27.

**Example 3:** Efficacy of 27DS in mouse infection model

3.1. Efficacy study in mouse sepsis model

*Pseudomonas aeruginosa* PAO1 was cultured in LB medium to mid-log phase and then washed 3 times with saline. *Pseudomonas aeruginosa* PAO1 $6.8 \times 10^6$ bacteria/mL suspended in saline was prepared.

0.2 mL *Pseudomonas aeruginosa* + 0.2 mL PBS
0.2 mL *Pseudomonas aeruginosa* + 0.2 mL EDTA (2 mM)
0.2 mL *Pseudomonas aeruginosa* + 0.2 mL 27DS (0.2 mg/mL)
0.2 mL *Pseudomonas aeruginosa* + 0.2 mL 27DS (0.2 mg/mL) + EDTA (2 mM)

The above mixtures were obtained, and 4 mL of each mixture was intraperitoneally administered to 6 mice in each group (0.1 mg/mL of 27DS and 1 mM of EDTA at the time of administration).

The survival of the mice was monitored for 2 days. Fig. 8 shows the results.

Next, a treatment experiment was conducted in *Pseudomonas* aeruginosa-infected mice.

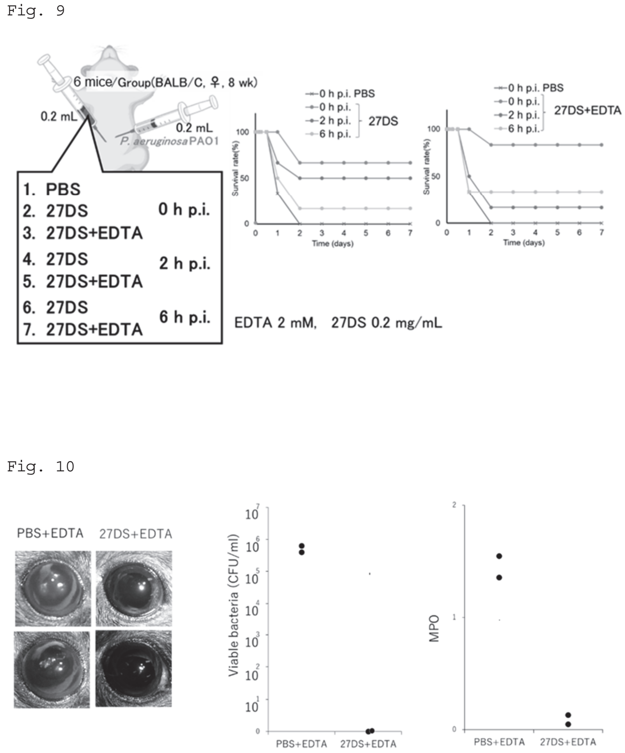

After inoculation, 0.2 mL of PBS or the following drugs were administered at 0, 2, and 6 hours. Fig. 9 shows the method and results.

27DS (0.2 mg/mL)
EDTA (2 mM)
27DS (0.2 mg/mL) + EDTA (2 mM)

3.2. Efficacy study in mouse keratitis model

*Pseudomonas aeruginosa* PAO1 was cultured in LB medium to mid-log phase and then washed 3 times with saline.

Mice were placed under general anesthesia using a combination of three anesthetic agents.

A bacterial suspension containing $5 \times 10^4$ cells/5 μL was prepared.

Three scratches were made and 5 μL of the bacterial suspension was instilled into the eye of each mouse.

After eye-drop instillation, the mice were immediately awakened with Antisedan.

After 30 minutes, the mice were held and 10 μl of the antibacterial enzyme 27DS (0.58 mg/ml) + EDTA (10 mM) was instilled into the eyes (only once).

After 24 hours, the mice were sacrificed.

The entire eyeballs were collected and disrupted in 500 μl of PBS. After dilution, 15 μl of the bacterial suspension was dropped into a square selective medium and cultured for 24 hours.

For measuring neutrophil myeloperoxidase (MPO), 125 μl was collected and measured without dilution. Fig. 10 shows the results.

The results reveal that 27DS + EDTA has efficacy against keratitis.

**Example 4:** Low incidence of resistant bacteria to 27DS

27DS was subcultured, and the minimum inhibitory concentration (MIC) was measured before and after culture. The measurement method was in accordance with Clinical & Laboratory Standards Institute (CLSI) Guidelines. Even after 20 subcultures, no changes were observed in the MICs of the bacterial strains, suggesting that 27DS-resistant bacteria were unlikely to emerge.

**Claims**

1. A polypeptide capable of permeating a lipopolysaccharide-containing outer membrane of Gram-negative bacteria, as defined in one of (i) to (iii) below:

   (i) a polypeptide comprising the amino acid sequence represented by SEQ ID NO: 1;
   (ii) a polypeptide comprising an amino acid sequence in which one or more amino acid residues are deleted, substituted, inserted, and/or added in the amino acid sequence represented by SEQ ID NO: 1; or
   (iii) a polypeptide comprising an amino acid sequence having sequence identity of 80% or higher with the amino acid sequence represented by SEQ ID NO: 1.

2. A polynucleotide as defined in one of (iv) to (vi) below:

   (iv) a polynucleotide comprising a DNA sequence encoding the polypeptide according to claim 1;
   (v) a polynucleotide comprising the DNA sequence represented by SEQ ID NO: 2; or
   (vi) a polynucleotide comprising a DNA sequence having sequence identity of 80% or higher with the polynucleotide comprising a DNA sequence as defined in (iv) or (v), and encoding a polypeptide capable of permeating a lipopolysaccharide-containing outer membrane of Gram-negative bacteria.

3. An antibacterial protein comprising an endolysin and the polypeptide according to claim 1, wherein the polypeptide according to claim 1 is located at the N-terminus or C-terminus of the endolysin.

4. A polynucleotide encoding the antibacterial protein according to claim 3.

5. An antibacterial agent or disinfectant against Gram-negative bacteria, comprising the antibacterial protein according to claim 3.

6. The antibacterial agent or disinfectant according to claim 5, wherein the Gram-negative bacteria are multidrug-resistant bacteria or *Pseudomonas aeruginosa.*

7. A pharmaceutical composition comprising the antibacterial protein according to claim 3.

8. The pharmaceutical composition according to claim 7, wherein a disease caused by the *Pseudomonas aeruginosa* is one or more diseases selected from the group consisting of keratitis, dacryocystitis, endophthalmitis, cellulitis-induced eye infections, sepsis, pneumonia, multidrug-resistant Gram-negative bacterial infections, opportunistic infections, and other *Pseudomonas* aeruginosa-induced diseases.

9. An antibacterial composition or disinfectant composition comprising:

   the antibacterial protein according to claim 3; and
   an outer membrane permeabilizer,

   wherein the outer membrane permeabilizer is ethylenediaminetetraacetic acid (EDTA) or a salt thereof.

10. The antibacterial composition or disinfectant composition according to claim 9, wherein the Gram-negative bacteria are multidrug-resistant bacteria or *Pseudomonas aeruginosa.*

11. A method for preventing a disease caused by a Gram-negative bacterial infection or for killing Gram-negative bacteria, the method comprising administering a pharmaceutical composition containing the antibacterial protein according to claim 3 to a human.

12. A method for preventing a disease caused by a Gram-negative bacterial infection or for killing Gram-negative bacteria, the method comprising administering a pharmaceutical composition containing the antibacterial protein according to claim 3 to an animal.

Fig. 1

Fig. 2

Fig. 3

PBS

EDTA

27DS

27DS+EDTA

Fig. 4

(A)

OD595

27DS

PBS

Increase in turbidity

Time (min)

(B)

**PBS treatment**    **27DS treatment**

Morphological changes
are induced in bacteria

Fig. 5

Fig. 6

*Pseudomonas aeruginosa* (27DS treatment)

*Pseudomonas aeruginosa* (27DS+EDTA treatment)

Fig. 7

Other bacterial strains (left: 27DS treatment; right: 27DS+EDTA treatment)

Fig. 8

Fig. 9

Fig. 10

## INTERNATIONAL SEARCH REPORT

| International application No. |
| --- |
| **PCT/JP2024/023622** |

| A. | CLASSIFICATION OF SUBJECT MATTER |
| --- | --- |

*C12N 15/62*(2006.01)i; *A01N 63/50*(2020.01)i; *A01P 3/00*(2006.01)i; *A61K 38/47*(2006.01)i; *A61K 47/18*(2017.01)i; *A61P 11/00*(2006.01)i; *A61P 27/02*(2006.01)i; *A61P 31/04*(2006.01)i; *C07K 14/005*(2006.01)i; *C07K 19/00*(2006.01)i; *C12N 15/12*(2006.01)i; *C12N 15/33*(2006.01)i

FI:  C12N15/62 Z; A61K47/18; A61P31/04; A61P27/02; A61P11/00; A61K38/47; A01N63/50; A01P3/00; C07K14/005; C12N15/33; C12N15/12; C07K19/00 ZNA

According to International Patent Classification (IPC) or to both national classification and IPC

| B. | FIELDS SEARCHED |
| --- | --- |

Minimum documentation searched (classification system followed by classification symbols)

C12N15/00-15/90; C07K1/00-19/00; A61K38/00-38/58; A61K47/00-47/69; A61P1/00-43/00; A01N1/00-65/48; A01P1/00-23/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); UniProt/GeneSeq

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | ORITO, Y. et al, Bacillus amyloliquefaciens phage endolysin can enhance permeability of Pseudomonas aeruginosa outer membrane and induce cell lysis. Appl Microbiol Biotechnol. 2004, vol. 65, pp. 105-109 | 1-8, 10-12 |
| | abstract, page 105, left column, line 22 to right column, line 18, page 108, right column, line 1 to page 109, left column, line 8, fig. 2, 3 | |
| Y | | 9 |
| Y | JP 2023-517245 A (TELUM THERAPEUTICS S.L.) 24 April 2023 (2023-04-24) claims, paragraphs [0078], [0079], [0110] | 9 |
| Y | WO 2022/158550 A1 (NATIONAL INSTITUTE OF ADVANCED INDUSTRIAL SCIENCE AND TECHNOLOGY) 28 July 2022 (2022-07-28) claims, paragraphs [0061], [0083] | 9 |

[✓] Further documents are listed in the continuation of Box C.    [✓] See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **16 July 2024** | **13 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2024/023622** |

### C. DOCUMENTS CONSIDERED TO BE RELEVANT

| Category* | Citation of document, with indication, where appropriate, of the relevant passages | Relevant to claim No. |
| --- | --- | --- |
| A | SHIN, S. Y. et al., Structure-Activity Analysis of SMAP-29, a Sheep Leukocytes-Derived Antimicrobial Peptide, Biochemical and Biophysical Research Communications, 2001, vol. 285, pp. 1046-1051<br>abstract, table 1 | 1-12 |
| A | DAWSON, R. M. et al., Analogues of peptide SMAP-29 with comparable antimicrobial potency and reduced cytotoxicity, International Journal of Antimicrobial Agents, 2011, vol. 37, pp. 432-437<br>abstract, table 1 | 1-12 |

Form PCT/ISA/210 (second sheet) (July 2022)

**INTERNATIONAL SEARCH REPORT**

International application No.

**PCT/JP2024/023622**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT
Information on patent family members

International application No.

**PCT/JP2024/023622**

| Patent document cited in search report | | | Publication date (day/month/year) | Patent family member(s) | | | Publication date (day/month/year) |
|---|---|---|---|---|---|---|---|
| JP | 2023-517245 | A | 24 April 2023 | US claims, paragraphs [0102], [0103], [0139]<br>EP | 2023/0138922<br><br>4118192 | A1<br><br>A1 | |
| WO | 2022/158550 | A1 | 28 July 2022 | US claims, paragraphs [0108], [0134] | 2024/0035001 | A1 | |

Form PCT/ISA/210 (patent family annex) (July 2022)

26

## REFERENCES CITED IN THE DESCRIPTION

**Patent documents cited in the description**

- JP 2021112152 A
- JP 2010536354 A
- JP 6261086 B

**Non-patent literature cited in the description**

- **LAI WCB** ; **CHEN X** ; **HO MKY** ; **XIA J** ; **LEUNG SSY**. Bacteriophage-derived endolysins to target Gram-negative bacteria. *Int J Pharm*, 2020, vol. 589, 119833
- **DE MAESSCHALCK V** ; **GUTIERREZ D** ; **PAE-SHUYSE J** ; **LAVIGNE R** ; **BRIERS Y**. Advanced engineering of third-generation lysins and formulation strategies for clinical applications. *Crit Rev Microbiol*, 2020, vol. 46 (5), 548-564
- **GONTIJO MTP** ; **JORGE GP** ; **BROCCHI M**. Current Status of Endolysin-Based Treatments against Gram-negative Bacteria. *Antibiotics*, 2021, vol. 10 (10), 1143
- *J Infect Dis.*, 2007, vol. 196 (8), 1237-47
- *Virus Research*, 2009, vol. 139 (1), 131-134
- *Viruses*, 2019, vol. 11 (9), 769
- *FEBS Letter*, 2001, vol. 500 (1-2), 56-59
- *mBio*, 01 July 2014, vol. 5 (4), e01379-14
- *Journal of the Japan Veterinary Medical Association*, 1976, vol. 29, 429-433